# FASCICULE DE BREVET EUROPEEN

(11) **EP 0 882 702 B1**
(45) Date de publication et mention de la délivrance du brevet: **04.10.2001**
(21) Numéro de dépôt: 98640002.6
(22) Date de dépôt: 20.05.1998
(51) Int. Cl.: C07C 233/47, C07C 231/02

(54) **Nouveau procédé de préparation de N-acylaminoacides**
Verfahren zur Herstellung von N-Acylaminosäuren
Process for the preparation of N-acylaminoacids

(30) Priorité: 01.06.1997 FR 9706846
(43) Date de publication de la demande: 09.12.1998
(73) Titulaire: LABORATOIRES ASEPTA, Monaco (Principauté) (MC)
(72) Inventeur: Dencausse, Laurent, 06000 Nice (FR); Artaud, Jacques, 06000 Nice (FR); Lacroix, Georges, 06000 Nice (FR); Clamou, Jean Luc, 06000 Nice (FR)
(74) Mandataire: Hautier, Jean-Louis

(56) Documents cités:
- WO-A-94/26694
- WO-A-96/28413
- FR-A- 2 676 741
- FR-A- 2 698 869
- GB-A- 886 753

## Description

La présente invention concerne un perfectionnement au procédé de préparation de N-acylaminoacides à partir de protéines qui sont hydrolysées pour donner des acides aminés, acides aminés qui sont ensuite acylés.

*Les acylaminoacides, et plus spécialement les lipoaminoacides, réalisés par acylation d'une chaîne grasse sur des acides aminés ou des peptides courts, obtenus par hydrolyse de protéines végétales ou animales, ont fait l'objet de nombreux travaux.*

*Le document FR-A-2.503.144 propose de nouveaux dérivés d'acides aminés obtenus par acylation de leurs fonctions acylables avec la chaîne butyryle, donnant ainsi des* *butyrylaminoacides. Les acides aminés utilisés pour l'obtention des butyrylaminoacides, peuvent être pris individuellement ou obtenus à partir d'hydrolysats totaux de protéines quelconques. L'invention s'étend également aux produits réalisés par la salification des fonctions carboxyliques de ces butyrylaminoacides, par des bases minérales, organiques ou biologiques, ou encore par des métaux du groupe oligo-éléments.*

*Le document FR-A-2.503.151 a pour objet de nouveaux dérivés d'acides aminés soufrés, résultant de l'acylation de leur fonction amine par une chaîne butyryle ; le carboxyle de l'acide aminé étant rendu actif, peut être libre ou salifié par des bases minérales, organiques ou biologiques, ou encore par des métaux du groupe oligo-éléments.*

*Le document FR-A-2.676.741 a trait à la réalisation de nouvelles structures lipopolyaminoacides et lipopeptides caractérisé en ce que les acides aminés ou les peptides courts issus de l'hydrolyse de protéines végétales, sont acylés à des chaînes grasses comportant de 4 à 30 atomes de carbone. Les protéines végétales hydrolysées par voie chimique ou enzymatique, sont des sous-produits de l'industrie des oléagineux, utilisé principalement sous forme de tourteaux, de concentrats ou d'isolats provenant des cultures de soja, colza, arachide, tournesol, lupin par exemple. Les acylats obtenus à partir des hydrolysats de protéines végétales conduisent à des structures dont les fonctions carboxyliques sont libres ou peuvent être salifiées par des agents alcalins, pour réaliser des détergents ou encore par des métaux.*
*Le document FR-A-2.698.869 expose la réalisation d'acylaminoacides caractérisés en ce qu'ils sont obtenus par acylation d'un hydrolysat total des chaînes polypeptidiques sécrétées par le BOMBYX MORI. Les acylaminoacides obtenus 0comportent des chaînes de 1 à 30 atomes de carbone acylées à la* *glycine, à l'alanine et à la sérine qui constituent 90% des acides aminés totaux des fibres de soie.*

Les synthèses décrites se déroulent en milieu aqueux. Elles mettent en jeu un hydrolysat de protéines animales ou végétales auquel est rajouté un agent acylant (chlorures d'acide, anhydrides d'acide,...). Le pH est maintenu entre 10 et 11 par l'ajout d'une solution aqueuse et d'un agent alcalin (soude, potasse,...).

L'étude analytique par chromatographie en phase liquide (colonne : silice 100 RP 18 ; phase mobile : méthanol/eau, 92/8, v/v) des mélanges après acylation montre que :
- la composition en N-acylaminoacides obtenue n'est pas l'image de la composition en acides aminés de la protéine de départ, et
- l'agent d'acylation hydrolysé est présent en quantité importante.

Le rendement de la réaction est inférieur à 50% et les proportions relatives de chaque N-acylaminoacide sont différentes de la teneur en acides aminés des protéines de départ.

Ces différences sont fonction de la nature des acides aminés présents dans les protéines de départ.

D'ailleurs, des expériences ont montré une différence très importante de réactivité des acides aminés vis-à-vis de l'agent acylant. La réactivité de la fonction amine vis-à-vis du carbonyle de l'agent acylant dépend de sa nucléophilie et respecte l'ordre glycine > alanine > sérine.

La même synthèse a été appliquée à un hydrolysat de fibroïne de soie (Bombyx mori) dont la composition molaire moyenne des quatre acides aminés principaux est : glycine 43%, alanine 32%, sérine 15%, tyrosine 12% (Chemistry and structure of silk, K. Komatsu, *Jpn. Agric. Res. Q.,* (1979), Vol. 13, N°1, 64-72). Les rendements obtenus en N-palmitoyl-aminoacides sont inférieurs à 50%. La composition moyenne en N-palmitoyl-aminoacides et en acide palmitique déterminée par chromatographie en phase liquide dans les conditions données ci-dessus est de : N-palmitoyl-glycine 30%, N-palmitoyl-alanine 10%, N-palmitoyl-sérine + tyrosine < 1% et acide palmitique 60%. Ces résultats montrent que les N-palmitoyl aminoacides ont une composition nettement différente, notamment pour les aminoacides hydroxylés (sérine et tyrosine) de la composition des acides aminés dans l'hydrolysat de départ et qu'ils sont associés à une quantité importante d'acide palmitique due à l'hydrolyse du chlorure de palmitoyle.

La présente invention a pour objet de réaliser des N-acylaminoacides à partir d'hydrolysats de protéines végétales ou animales dont les compositions respectent la composition en acides aminés des protéines de départ et qui, de plus, sont exempts de produit d'hydrolyse de l'agent acylant.

La stratégie de synthèse consiste à travailler en milieu anhydre afin d'éliminer toute hydrolyse du réactif acylant et d'utiliser des dérivés d'acides aminés (esters) solubles en milieu organique pour réaliser la condensation de l'agent acylant.

A cet effet, l'invention concerne un procédé de préparation de N-acylaminoacides à partir de protéines qui sont hydrolysées pour donner des acides aminés, caractérisé en ce que les réactions sont les suivantes :
1) l'estérification de chaque acide aminé en présence d'alcool,
2) l'acylation de l'ester en présence d'un halogénure d'acide, et
3) la saponification du N-acylaminoacide ester en présence d'une base pour donner le N-acylaminoacide.

L'invention concerne également un procédé de préparation de N-acylaminoacide ester à partir de protéines qui sont hydrolysées pour donner des acides aminés, caractérisé en ce que les réactions sont les suivantes :
1) l'estérification de chaque acide aminé en présence d'alcool, et
2) l'acylation de l'ester en présence d'un halogénure d'acide.

Dans tous les cas, l'acylation s'effectue en milieu anhydre.

Dans le cas d'une synthèse de N-acylaminoacide, les réactions sont les suivantes : 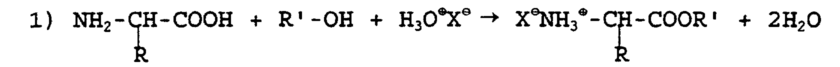 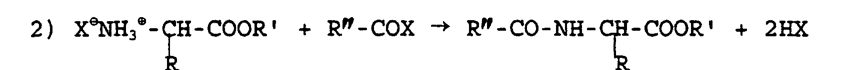 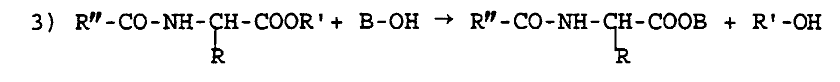 et 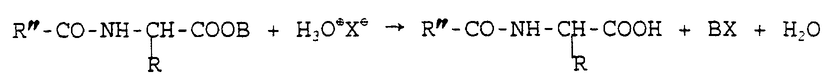 où R, R' et R" sont des radicaux alkyles ou aryles, saturés ou non, substitués ou non, B est un cation alcalin ou terreux et X est un anion halogène, sulfate ou phosphate.

Dans le cas d'une synthèse de N-acylaminoacide ester, les réactions sont les suivantes : 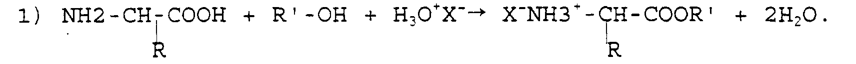 et

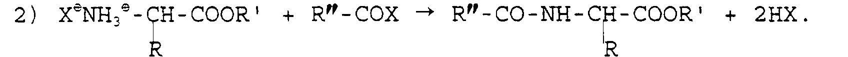

L'estérification s'effectue en milieu alcoolique par 1,2 à 1,8 équivalent d'acide chlorhydrique ou autre.

L'acylation s'effectue stoechiométriquement entre le chlorure d'acide et la fonction amine de chaque acide aminé estérifié.

L'acylation se déroule en présence de tous solvants aprotiques, tels le dichlorométhane ou le chloroforme, des esters d'acides aminés et des chlorures d'acides, et/ou en présence de deux à trois équivalents de triéthylamine ou de pyridine ou d'une base organique.

L'acylation s'effectue à une température comprise entre 0 et 5°C, sous agitation constante pendant 1 à 2 heures, puis le mélange réactionnel est lavé à neutralité pour éliminer la base organique.

La phase organique de la réaction d'acylation est séchée puis évaporée sous vide.

La saponification s'effectue en présence d'une base, telle que soude ou potasse, de concentration comprise entre 0,5 à 4 M, en milieu aqueux à reflux pendant 1 à 2 heures.

Le mélange réactionnel, lors de la saponification, est acidifié à un pH compris entre 2 et 3 par une solution d'acide chlorhydrique concentré à environ 6 M.

Après la saponification, le précipité de N-acylaminoacide est filtré, lavé puis séché.

Les N-acylaminoacides sont des lipoaminoacides.

Les N-acylaminoacides esters sont des lipoaminoacides esters.

Les protéines sont constituées par la fibroïne de soie (Bombyx mori).

Selon une variante, les protéines sont issues de crin de cheval (Equus caballus).

La synthèse se déroule en quatre étapes.

### 1. Hydrolyse des protéines :

Les protéines sont hydrolysées par de l'acide chlorhydrique concentré (10-12 M) pendant plusieurs heures à reflux jusqu'à l'obtention d'une réaction biurétique négative. Le mélange réactionnel est neutralisé (pH=5-7) par de la soude concentrée (environ 10 M). En cas de coloration, il est traité à chaud (75-80°C) par du charbon actif pendant une heure.

### 2. Synthèse des esters des acides aminés des hydrolysats :

Les hydrolysats neutralisés et décolorés sont évaporés à sec. La poudre obtenue est un mélange d'acides aminés et de sel. Le nombre de moles d'acides aminés est déterminé par un dosage de l'azote aminé suivant Sörensen. Ce mélange est traité en milieu alcoolique (alcool) par 1,2 à 1,8 équivalents d'acide chlorhydrique ou de chlorure de thionyle ou plus généralement par tous réactifs (HX) susceptibles de préparer les esters des acides aminés selon la réaction 1 : 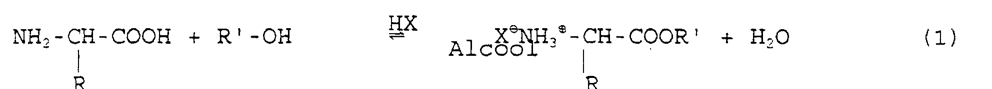 Le rendement de la réaction est de 96-98%.

### 3. Synthèse des N-acylaminoacides estérifiés :

L'agent d'acylation, constitué par exemple de chlorures d'acides, réagit stoechiométriquement selon la réaction 2 avec les fonctions amines des acides aminés estérifiés. La réaction se déroule dans le dichlorométhane ou le chloroforme ou plus généralement dans tous solvants aprotiques (solvant) des esters d'acides aminés et de l'agent d'acylation en présence de 2 à 3 équivalents de triéthylamine ou de pyridine ou d'une base organique (base) afin de neutraliser l'acide libéré. 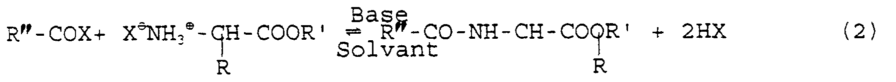 R" est une chaîne grasse comportant 3 à 29 atomes de carbone.

La réaction est effectuée à la température de 0-5°C. Le mélange est ensuite maintenu sous agitation pendant une à deux heure(s) à cette température puis lavé à neutralité pour éliminer la base organique. La phase organique est séchée puis évaporée sous vide.

Le rendement de la réaction est de 90-95%.

### 4. Synthèse des N-acylaminoacides :

Les N-acylaminoacides esters sont saponifiés (réaction 3) par action d'une base (BOH) à 0,5 à 4 M (soude, potasse,...) en milieu aqueux à reflux pendant une à deux heures.

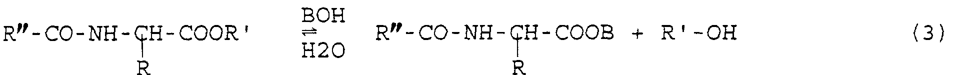

Le mélange réactionnel est acidifié à pH 2-3 par une solution d'acide chlorhydrique concentré à environ 6 M (réaction 4). Le précipité de N-acylaminoacides est filtré, lavé puis séché.

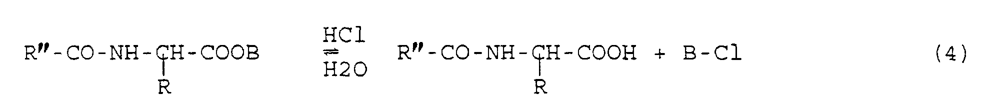

Le rendement de la réaction est de 97-99%.

L'étude analytique par chromatographie en phase liquide (colonne : silice 100 RP 18 ; phase mobile : méthanol/eau, 92/8, v/v) des N-acylaminoacides sous forme estérifiée montre que :
- la composition en aminoacides de la protéine de départ est respectée, et
- l'agent d'acylation hydrolysé est absent.

Exemples de synthèses de N-acylaminoacides selon l'invention.

### Exemple 1 : Synthèse de N-palmitoyl-aminoacides à partir de fibroïne de soie

### 1. Préparation de la fibroïne de soie :

La matière première de départ est constituée de déchets de cocons de vers à soie qui sont des rebuts de l'industrie de la soie. Ces cocons sont dans un premier temps triés afin d'éliminer les chrysalides résiduelles.

Les cocons vides (100 g) sont ensuite décreusés par lavages successifs dans une solution de carbonate de sodium à 10% et d'eau savonneuse (10 g/l). Les solutions sont portées dans les deux cas à ébullition pendant une heure. Ces opérations (2 lavages successifs) éliminent la séricine (grès) qui entoure la fibroïne et donne la structure au cocon. La fibroïne ainsi obtenue représente 65 g.

Le rendement du traitement est de 65%.

### 2. Hydrolyse de la fibroïne de soie :

Dans un ballon bicol de 1 litre sont additionnés 50 g de fibroïne de soie et 400 ml d'acide chlorhydrique concentré (12 M). Le mélange est chauffé à reflux pendant 4 heures (réaction biurétique négative) puis neutralisé à pH 6-7 à l'aide d'une solution de soude 12 M.

Le mélange de couleur marron foncée est ensuite chauffé à 75-80°C en présence de charbon actif (8%, m/m) pendant une heure.

L'hydrolysat est débarrassé des impuretés et du charbon actif par filtration sur filtre millipore sous pression réduite. Ainsi, la solution aqueuse, contenant les acides aminés et le chlorure de sodium, est éclaircie (couleur beige clair).

L'eau est éliminée à 50°C sous pression réduite. L'hydrolysat est séché sous vide. La masse obtenue est de 218 g. Ce mélange contient (Sörensen) 0,436 mole d'acides aminés.

### 3. Estérification de l'hydrolysat :

Placer dans un ballon bicol de 1 litre, les 218 g d'hydrolysat et 400 ml de méthanol. Ajouter goutte à goutte, sous agitation, 1,5 équivalents (0,65 mole) de chlorure de thionyle (48 ml ou 78,5 g) à l'aide d'une ampoule à verser. Le mélange réactionnel est maintenu entre 15 et 20°C. Lorsque la totalité du chlorure de thionyle est additionné, le mélange est porté à reflux pendant une heure. Le mélange est filtré sous vide et le méthanol est évaporé. Le mélange est séché sous vide pendant 12 heures.

Les esters d'acides aminés se présentent sous la forme d'une pâte visqueuse marron clair. La masse obtenue est de 57 g.

Le rendement par rapport aux acides aminés est de 96%.

### 4. Synthèse des esters de N-palmitoyl-aminoacides de fibroïne de soie :

Les 57 g d'esters d'aminoacides sous forme de chlorhydrates sont placés dans un ballon bicol de 1 litre en présence de 500 ml de dichlorométhane et de 146 ml de triéthylamine (2,5 équivalents/acides aminés estérifiés).

Le mélange est refroidi entre 0-5°C puis additionné de 114,5 ml (126,5 g) de chlorure de palmitoyle (0,9 équivalent/acides aminés estérifiés) en solution dans 30 ml de dichlorométhane. Après addition du chlorure de palmitoyle, le mélange qui présente un aspect laiteux est agité pendant une heure puis transféré dans une ampoule à décanter. La phase organique est lavée par 4 fois 250 ml d'eau acidulée pour éliminer la triéthylamine en excès puis séchée sur du sulfate de magnésium anhydre. La phase organique séchée est ensuite filtrée et évaporée sous vide. On obtient 122,5 g de N-palmitoyl-aminoacides de fibroïne de soie estérifiés (Tf=69-70°C) qui présente une couleur beige clair.

Le rendement de la synthèse est de 86%.

### 5. Synthèse des N-palmitoyl-aminoacides de fibroïne de soie :

Les 122,5 g d'esters méthyliques de N-palmitoyl-aminoacides de fibroïne de soie sont introduits dans un ballon de 1 litre et additionnés de 400 ml d'une solution aqueuse de soude 1 M (1,2 équivalents ou 0,4 mole). Le mélange est chauffé à reflux pendant une heure. La solution aqueuse limpide, contenant les sels de sodium des N-palmitoyl-aminoacides, est transférée dans un erlenmeyer de 2 litres et acidifiée à pH 1,5 avec une solution d'acide chlorhydrique 8 M. Le mélange est homogénéisé grâce à une agitation mécanique puis additionné de 500 ml d'eau à 0°C, ce qui favorise la décantation des N-palmitoyl-aminoacides. Les N-palmitoyl-aminoacides sont filtrés sur büchner, rincés avec de l'eau glacée et séchés sous vide. La masse obtenue est de 104 g. Le point de fusion des N-palmitoyl-aminoacides de fibroïne de soie de 92-98°C.

Le rendement est de 88%.

L'étude analytique par chromatographie en phase liquide (colonne : silice 100 RP 18 ; phase mobile : méthanol/eau, 92/8, v/v) des N-palmitoyl-aminoacides sous forme estérifiée montre que :
- la composition en aminoacides de la protéine de départ est respectée : N-palmitoyl-glycine 50%, N-palmitoyl-alanine 25%, N-palmitoyl-sérine + tyrosine 21%, N-palmitoyl-valine 2% et N-palmitoyl-thréonine 2%, et
- l'acide palmitique (agent d'acylation hydrolysé) est absent.

### Exemple 2 : Synthèse de N-palmitoyl-aminoacides à partir de crin de cheval

### 1. Préparation du crin de cheval :

La matière première de départ est constituée de crin de cheval brut qui est délipidé par de l'hexane.

100 g de crin de cheval sont extraits par de l'hexane dans un soxhlet pendant 6 heures. Le rendement de l'opération est de 98%.

### 2. Hydrolyse du crin de cheval :

Les 62 g de crin de cheval sont placés dans un ballon bicol de 1 litre et additionnés de 400 ml d'acide chlorhydrique concentré (12M). Le mélange est chauffé à reflux pendant 4 heures (réaction biurétique négative) puis neutralisé à pH 5 à l'aide d'une solution de soude de 12 M.

Le mélange de couleur marron foncée est ensuite chauffé à 75-80°C en présence de charbon actif (8%, m/m) pendant une heure).

L'hydrolysat est débarrassé des impuretés et du charbon actif par filtration sur filtre millipore sous pression réduite. L'eau est éliminée à 50°C sous pression réduite. L'hydrolysat est séché sous vide. La masse obtenue est de 221 g. Ce mélange contient (Sörensen) 0,335 mole d'acides aminés.

### 3. Estérification de l'hydrolysat :

Placer dans un ballon bicol de 1 litre, les 221 g d'hydrolysat et 400 ml de méthanol. Ajouter goutte à goutte, sous agitation, 1,5 équivalents (0,5 mole) de chlorure de thionyle (36,7 ml ou 59,8 g) à l'aide d'une ampoule à verser. Le mélange réactionnel est maintenu entre 15 et 20°C. Lorsque la totalité du chlorure de thionyle est additionnée, le mélange est porté à reflux pendant une heure. Le mélange est filtré sous vide et le méthanol est évaporé. Le mélange est séché sous vide pendant 12 heures.

Les esters d'acides aminés se présentent sous la forme d'une pâte visqueuse marron clair. La masse obtenue est de 86,5 g.

Le rendement par rapport aux acides aminés est de 96%.

### 4. Synthèse des esters de N-palmitoyl-aminoacides de crin de cheval :

Les 86,5 g d'esters d'aminoacides sous forme de chlorhydrates sont placés dans un ballon bicol de 1 litre en présence de 500 ml de dichlorométhane et de 112 ml de triéthylamine (2,5 équivalents/acides aminés estérifiés).

Le mélange est refroidi entre 0-5°C puis additionné de 89,5 ml (81 g) de chlorure de palmitoyle (0,9 équivalent/acides aminés estérifiés) en solution dans 30 ml de dichlorométhane. Après addition du chlorure de palmitoyle, le mélange qui présente un aspect laiteux est agité pendant une heure puis transféré dans une ampoule à décanter. La phase organique est lavée par 4 fois 250 ml d'eau acidulée pour éliminer la triéthylamine en excès puis séchée sur du sulfate de magnésium anhydre. La phase organique séchée est ensuite filtrée et évaporée sous vide. On obtient 136 g de N-palmitoyl-aminoacides de crin de cheval estérifiés (Tf= 44-45°C) qui présente une couleur beige clair.

Le rendement de la synthèse est de 86%.

### 5. Synthèse des N-palmitoyl-aminoacides de crin de cheval :

Les 136 g d'ester méthyliques de N-palmitoyl-aminoacides de crin de cheval sont introduits dans un ballon de 1 litre et additionnés de 400 ml d'une solution aqueuse de soude de 1 M (1,2 équivalents ou 0,4 mole). Le mélange est chauffé à reflux pendant une heure. La solution aqueuse limpide contenant les sels de sodium des N-palmitoyl-aminoacides est transférée dans un erlenmeyer de 2 litres et acidifiée à pH 1,5 avec une solution d'acide chlorhydrique 8 M. Le mélange est homogénéisé grâce à une agitation mécanique puis additionné de 500 ml d'eau à 0°C ce qui favorise la décantation des N-palmitoyl-aminoacides. Les N-palmitoyl-aminoacides sont filtrés sur büchner, rincés avec de l'eau glacée et séchés sous vide. La masse obtenue est de 112 g. Le point de fusion des N-palmitoyl-aminoacides de crin de cheval est 60-68°C.

Le rendement est de 88%.

L'étude analytique par chromatographie en phase liquide (colonne : silice 100 RP 18 ; phases mobiles : (1) méthanol/eau, 92/8, v/v ; (2) méthanol pour l'analyse du N-palmitoyl-cystéine) donne la composition des N-palmitoyl-aminoacides sous forme estérifiée ci-dessous :
- N-palmitoyl-glycine + N-palmitoyl-acide aspartique + N-palmitoyl-acide glutamique 33%, N-palmitoyl-sérine + tyrosine 26%, N-palmitoyl-leucine 9%, N-palmitoyl-cystéine 8%, N-palmitoyl-alanine 7%, N-palmitoyl-thréonine 5%, N-palmitoyl-valine 2%.

Cette composition est proche de la composition moyenne en aminoacides du crin de cheval (Studies on the amino acid compositions of the equine bodyhair and the hoof, T. Samata et M. Matsuda Jpn. J. Vet. Sci., (1988), 50, 330-340).

L'acide palmitique (agent d'acylation hydrolysé) est absent.

La présente invention a donc pour objet de réaliser des structures lipoaminoacides, qui sont synthétisées en milieu anhydre, à une température se situant entre 0 et 5°C, conduisant à un rendement en lipoaminoacides supérieure à 70% par rapport à la protéine de départ.

Une autre caractéristique importante, par rapport à l'état de la technique, réside dans le fait que le produit obtenu est exempt d'acides gras libres.

Parmi les protéines de base pour ces réactions, la plus intéressante est la kératine.

Ainsi, la fibroïne de la soie est une kératine β qui contient quatre acides aminés principaux (glycine, alanine, sérine et tyrosine) et qui est particulièrement intéressante.

De même, le crin de cheval est une kératine α qui contient les mêmes acides aminés que précédemment plus de la cystéine et de la cystine.

L'intérêt de la kératine réside dans la proportion assez importante d'acides aminés hydroxylés (sérine et tyrosine), les N-acylaminoacides hydroxylés ayant un effet très positif dans l'hydratation de la peau.

## Revendications

1. Procédé de préparation de N-acylaminoacides à partir de protéines, par hydrolyse de ces protéines pour donner des acides aminés, estérification de chaque acide aminé en présence d'alcool, acylation de l'ester en présence d'un halogénure d'acide, et saponification du N-acylaminoacide ester en présence d'une base pour donner le N-acylaminoacide, **caractérisé en ce que** la réaction d'acylation s'effectue en milieu anhydre.

2. Procédé de préparation de N-acylaminoacides ester à partir de protéines, par hydrolyse de ces protéines pour donner des acides aminés, estérification de chaque acide aminé en présence d'alcool, acylation de l'ester en présence d'un halogénure d'acide, **caractérisé en ce que** la réaction d'acylation s'effectue en milieu anhydre.

3. Procédé, selon la revendication 1, **caractérisé en ce que** les réactions sont les suivantes : 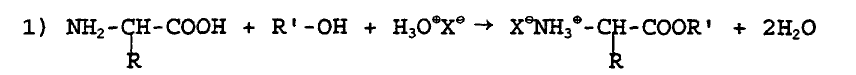 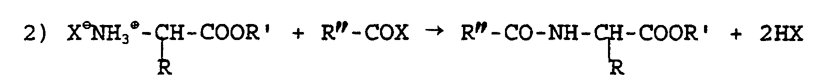 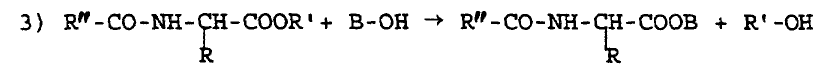 et 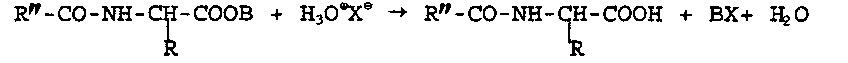 où R, R' et R" sont des radicaux alkyles ou aryles, saturés ou non, substitués ou non, B est un cation alcalin ou terreux et X est un anion halogène, sulfate ou phosphate.

4. Procédé, selon la revendication 2, **caractérisé en ce que** les réactions sont les suivantes : 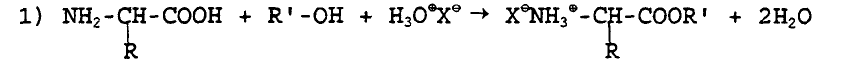 et

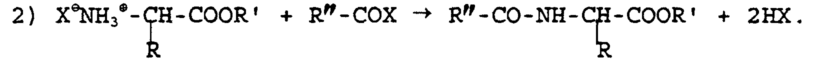

5. Procédé, selon l'une quelconque des revendications 1, 2, 3 ou 4, **caractérisé en ce que** l'estérification s'effectue en milieu alcoolique par 1,2 à 1,8 équivalent d'acide chlorhydrique ou autre.

6. Procédé, selon l'une quelconque des revendications 1, 2,3 ou 4, **caractérisé en ce que** l'acylation s'effectue stoechiométriquement entre le chlorure d'acide et la fonction amine de chaque acide aminé estérifié.

7. Procédé, selon l'une quelconque des revendications 1, 2, 3, 4 ou 6, **caractérisé en ce**
**que** l'acylation se déroule en présence de tous solvants aprotiques, tels le dichlorométhane ou le chloroforme, des esters d'acides aminés et des chlorures d'acides, et/ou en présence de deux à trois équivalents de triéthylamine ou de pyridine ou d'une base organique.

8. Procédé, selon l'une quelconque des revendications 1, 2, 3, 4, 6 ou 7, **caractérisé en ce**
**que** l'acylation s'effectue à une température comprise entre 0 et 5°C, sous agitation constante pendant 1 à 2 heures, puis le mélange réactionnel est lavé à neutralité pour éliminer la base organique.

9. Procédé, selon l'une quelconque des revendications 1, 2, 3, 4 ou 6 à 8, **caractérisé en ce que** la phase organique de la réaction d'acylation est séchée puis évaporée sous vide.

10. Procédé, selon l'une quelconque des revendications 1 ou 3, **caractérisé en ce**
**que** la saponification s'effectue en présence d'une base, telle que soude ou potasse, de concentration comprise entre 0,5 à 4 M, en milieu aqueux à reflux pendant 1 à 2 heures.

11. Procédé, selon l'une quelconque des revendications 1, 3 ou 10, **caractérisé en ce**
**que** le mélange réactionnel, lors de la saponification, est acidifié à un pH compris entre 2 et 3 par une solution d'acide chlorhydrique concentré à environ 6 M.

12. Procédé, selon l'une quelconque des revendications 1, 3, 10 ou 11, **caractérisé en ce**
**qu'**après la saponification, le précipité de N-acylaminoacide est filtré, lavé puis séché.

13. Procédé, selon l'une quelconque des revendications 1, 3 ou 12, **caractérisé en ce**
**que** les N-acylaminoacides sont des lipoaminoacides.

14. Procédé, selon l'une quelconque des revendications 1, 2, 3, 5 ou 12, **caractérisé en ce**
**que** les N-acylaminoacides esters sont des lipoaminoacides esters.

15. Procédé,selon l'une quelconque des revendications 1 ou 2, **caractérisé en ce**
**que** les protéines sont constituées à partir de sources naturelles, animales ou végétales.

16. Procédé, selon l'une quelconque des revendications 1 ou 2 ou 15, **caractérisé en ce**
**que** les protéines sont constituées par la fibroine de soie (Bombyx mori).

17. Procédé, selon l'une quelconque des revendications 1 ou 2 ou 15, **caractérisé en ce**
**que** les protéines sont issues de crin de cheval (Equus caballus).

## Claims

1. Process for preparation of N-acylaminoacids from proteins, by hydrolysis of these proteins to give amino acids, esterification of each amino acid in the presence of alcohol, acylation of ester in the presence of an acid halide, and saponification of ester N-acylaminoacid in the presence of a base to give N-acylaminoacid,
**characterized in that** the acylation reaction is carried out in an anhydrous medium.

2. Process for preparation of ester N-acylaminoacids from proteins, by hydrolysis of these proteins to give amino acids, esterification of each amino acid in the presence of alcohol, acylation of ester in the presence of an acid halide,
**characterized in that** the acylation reaction is carried out in an anhydrous medium.

3. Process, according to claim 1, **characterized in that** the reactions are as follows 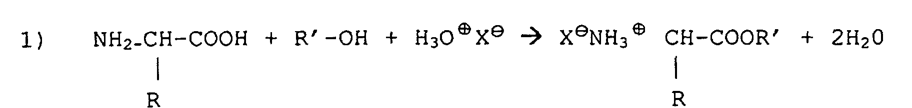 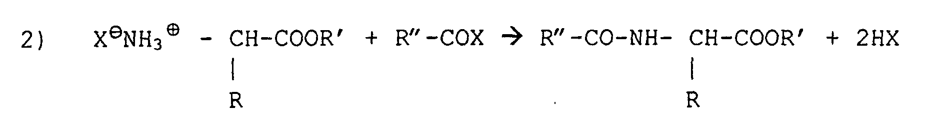 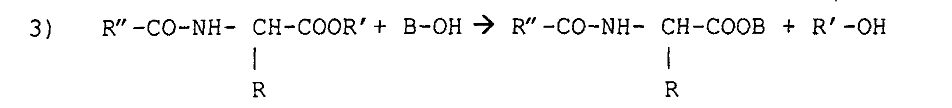 and 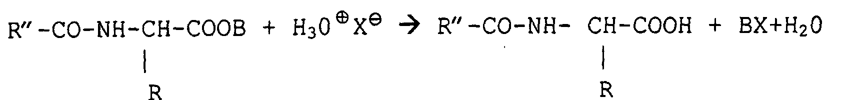 where R, R' et R" are alkyls or aryls radicals, saturated or not, substituted or not, B is an alkaline or earthy cation and X is a halogenous, sulphate or phosphate anion.

4. Process, according to claim 2, **characterized in that** the reactions are as follows 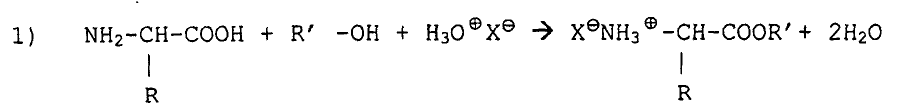 and

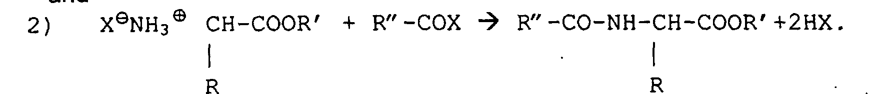

5. Process, according to any of claims 1, 2, 3 or 4, **characterized in that** esterification is carried out in an alcoholic medium by 1.2 to 1.8 hydrochloric or different acid equivalent.

6. Process, according to any of claims 1, 2, 3 or 4, **characterized in that** the acylation is carried out stoichiometrically between the acid chloride and the amine function of each esterified amino acid.

7. Process, according to any of claims 1, 2, 3, 4 or 6, **characterized in that** the acylation takes place in the presence of any aprotic solvent, such as dichloromethane or chloroform, amino acid esters and acid chlorides, and/or in the presence of two to three equivalents of triethylamine or pyridine or of an organic base.

8. Process, according to any of claims 1, 2, 3, 4, 6 or 7, **characterized in that** the acylation is carried out at a temperature ranging between O and 5°C, with constant agitation during 1 to 2 hours, then the reaction mixture is washed to neutrality in order to eliminate the organic base.

9. Process, according to any of claims 1, 2, 3, 4 or 6 to 8, **characterized in that** the organic phase of the acylation reaction is dried then evaporated in a vacuum.

10. Process, according to any of the claims 1 or 3, **characterized in that** saponification is carried out in the presence of a base, such as soda or potash, with concentration ranging between 0,5 to 4 M, in aqueous medium with reversed flow during 1 to 2 hours.

11. Process, according to any of claims 1, 3 or 10, **characterized in that** the reaction mixture, during saponification, is rendered more acid with a pH ranging between 2 and 3 by a hydrochloric acid solution concentrated to approximately 6 M.

12. Process, according to any of claims 1, 3, 10 or 11, **characterized in that** after saponification, the N-acylaminoacid precipitate is filtered, washed then dried.

13. Process, according to any of claims 1, 3 or 12, **characterized in that** the N-acylaminoacids are lipoaminoacids.

14. Process, according to any of claims 1, 2, 3, 5 or 12, **characterized in that** the ester N-acylaminoacids are ester lipoaminoacids.

15. Process, according to any of the claims 1 or 2, **characterized in that** the proteins are constituted from natural, animal or plant sources.

16. Process, according to any of claims 1 or 2 or 15, **characterized in that** the proteins are constituted from the silk fibroin (Bombyx Mori).

17. Process, according to any of claims 1 or 2 or 15,
**characterized in that** the proteins are obtained from horse hair (Equus caballus).

## Patentansprüche

1. Verfahren zur Herstellung von N-Acylaminosäuren, ausgehend von Proteinen, durch Hydrolyse dieser Proteinen, um Aminosäuren zu ergeben, Veresterung jeder Aminosäure in Anwesenheit von Alkohol, Acylation des Esters in Anwesenheit eines Säurehalogenids, und Verseifung der Ester-N-Acylaminosäure in Anwesenheit einer Base, um die N-Acylaminosäure zu ergeben, **dadurch gekennzeichnet, daß** die Acylation in wasserfreiem Medium erfolgt.

2. Verfahren zur Herstellung von Ester-N-Acylaminosäuren, ausgehend von Proteinen, durch Hydrolyse dieser Proteinen, um Aminosäuren zu ergeben, Veresterung jeder Aminosäure in Anwesenheit von Alkohol, Acylation des Esters in Anwesenheit eines Säurehalogenids, **dadurch gekennzeichnet, daß** die Acylierung in wasserfreiem Medium erfolgt.

3. Verfahren gemäß Anspruch 1, **dadurch gekennzeichnet, daß** die Reaktionen die folgenden sind: 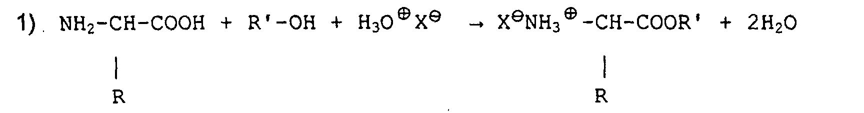 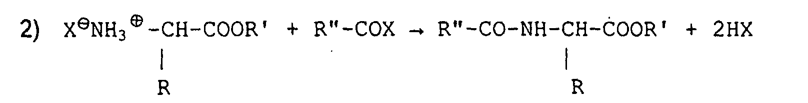 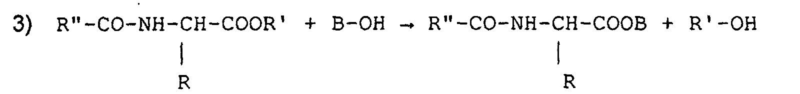 und 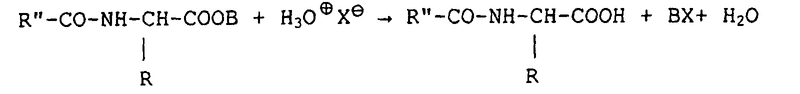 wobei R, R' und R" gesättigte oder ungesättigte, substituierte oder nicht substituierte Alkyl-oder Arylradikale sind, B ein alkalisches oder erdiges Kation, und X ein Halogen-, Sulfat-oder Phosphat-Anion ist.

4. Verfahren gemäß Anspruch 2, **dadurch gekennzeichnet, daß** die Reaktionen die folgenden sind: 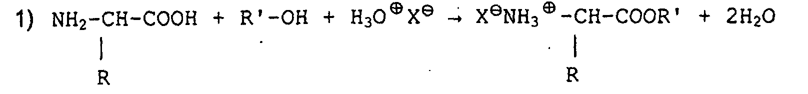 und

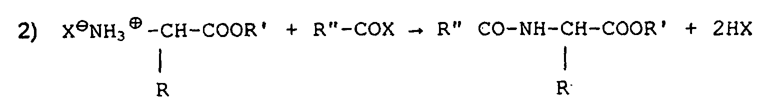

5. Verfahren gemäß irgendeinem der Ansprüche 1, 2, 3 oder 4, **dadurch gekennzeichnet, daß** die Veresterung in alkoholischem Medium mit 1,2 bis 1,8 äquivalenter Chlorwasserstoff- oder sonstiger Säure erfolgt.

6. Verfahren gemäß irgendeinem der Ansprüche 1, 2, 3 oder 4, **dadurch gekennzeichnet, daß** die Acylation stöchiometrisch zwischen dem Säurechlorid und der Aminfunktion jeder veresterten Aminosäure erfolgt.

7. Verfahren gemäß irgendeinem der Ansprüche 1, 2, 3, 4 oder 6, **dadurch gekennzeichnet, daß** die Acylation in Anwesenheit von jeglichem aprotischen Lösungsmittel, wie Dichlormethan oder Chloroform, von Aminosäure-Estern und von Säurechloriden, und/oder in Anwesenheit von zwei bis drei Äquivalenten von Triäthylamin oder von Pyridin oder einer organischen Base abläuft.

8. Verfahren gemäß irgendeinem der Ansprüche 1, 2, 3, 4, 6 oder 7, **dadurch gekennzeichnet, daß** die Acylation bei einer Temperatur zwischen 0 und 5°C unter konstantem Rühren während 1 bis 2 Stunden erfolgt, dann die reaktionelle Mischung zu Neutralität gewaschen wird, um die organische Base zu eliminieren.

9. Verfahren gemäß irgendeinem der Ansprüche 1, 2, 3, 4 oder 6 bis 8, **dadurch gekennzeichnet, daß** die organische Phase der Acylationsreaktion getrocknet und dann in Vacuum verdampft wird.

10. Verfahren gemäß irgendeinem der Ansprüche 1 oder 3, **dadurch gekennzeichnet, daß** die Verseifung in Anwesenheit einer Base, wie z.B. Soda oder Kaliumkarbonat, mit einer Konzentration zwischen 0,5 und 4 M in wasserigem Medium mit Rücklauf während 1 bis 2 Stunden erfolgt.

11. Verfahren gemäß irgendeinem der Ansprüche 1, 3 oder 10, **dadurch gekennzeichnet, daß** die reaktionelle Mischung bei der Verseifung mittels einer zu ca. 6 M konzentrierten Hydrochlorsäure-Lösung zu einem pH zwischen 2 und 3 sauer gemacht wird.

12. Verfahren gemäß irgendeinem der Ansprüche 1, 3, 10 oder 11, **dadurch gekennzeichnet, daß** nach der Verseifung der Niederschlag von N-Acylaminosäure gefiltert, gewaschen und dann getrocknet wird.

13. Verfahren gemäß irgendeinem der Ansprüche 1, 3 oder 12, **dadurch gekennzeichnet, daß** die N-Acylaminosäuren Lipoaminosäuren sind.

14. Verfahren gemäß irgendeinem der Ansprüche 1, 2, 3, 5 oder 12, **dadurch gekennzeichnet, daß** die Ester-N-Acylaminosäuren Ester-Lipoaminosäuren sind.

15. Verfahren gemäß irgendeinem der Ansprüche 1 oder 2, **dadurch gekennzeichnet, daß** die Proteine aus natürlichen, tierischen oder pflanzlichen, Quellen gebildet werden.

16. Verfahren gemäß irgendeinem der Ansprüche 1 oder 2 oder 15, **dadurch gekennzeichnet, daß** die Proteine von Seide-Fibroin (Bombyx mori) gebildet werden.

17. Verfahren gemäß irgendeinem der Ansprüche 1 oder 2 oder 15, **dadurch gekennzeichnet, daß** die Proteine aus Roßhaar (Equus caballus) stammen.
